Europäisches Patentamt

European Patent Office

Office européen des brevets

(11) Numéro de publication : **0 448 464 A1**

(12) # DEMANDE DE BREVET EUROPEEN

(21) Numéro de dépôt : **91400748.9**

(51) Int. Cl.⁵ : **G01N 33/68, C12N 5/08, C07K 15/06, C07K 15/28, A61K 37/02, A61B 17/425**

(22) Date de dépôt : **20.03.91**

(30) Priorité : 20.03.90 FR 9003537

(43) Date de publication de la demande :
**25.09.91 Bulletin 91/39**

(84) Etats contractants désignés :
**AT BE CH DE DK ES FR GB GR IT LI LU NL SE**

(71) Demandeur : **SOCIETE DE RECHERCHE ET DE DEVELOPPEMENT EN ACTIVATION ET COMMUNICATION CELLULAIRE**
**La Musardière, Via Aurelia**
**F-06610 La Gaude (FR)**

(72) Inventeur : **Fenichel, Patrick**
**170 Chemin de l'Adret**
**F-06610 La Gaude (FR)**
Inventeur : **Hsi, Bae-Li**
**18 Domaine de l'Aspre**
**F-06790 Aspremont (FR)**

(74) Mandataire : **Ahner, Francis et al**
**CABINET REGIMBEAU, 26, avenue Kléber**
**F-75116 Paris (FR)**

(54) **Procédé pour la mise en évidence de la capacité d'un spermatozoide à subir la réaction acrosomale.**

(57)    L'invention concerne un procédé de mise en évidence de la capacité d'un spermatozoïde contenu dans un milieu donné à subir la réaction acrosomale.

Il est caractérisé en ce que, sur ledit spermatozoïde sur lequel on induit la réaction acrosomale, on met en évidence qualitativement et/ou quantitativement la fixation des protéines C3b ou iC3 ou d'un fragment desdites protéines ajoutées au milieu contenant ledit spermatozoïde.

Application à la réalisation de spermogrammes et à la préparation de milieux de culture convenant pour la fécondation in vitro.

EP 0 448 464 A1

# PROCEDE POUR LA MISE EN EVIDENCE DE LA CAPACITE D'UN SPERMATOZOÏDE A SUBIR LA REACTION ACROMOSALE

L'invention a pour objet un procédé de mise en évidence de la capacité d'un spermatozoïde à subir la réaction acrosomale et l'utilisation de ce procédé pour la mise au point d'un milieu optimal pour les fécondations in vitro.

La capacitation et la réaction acrosomale sont deux étapes successives indispensables pour qu'un spermatozoïde humain soit fécond.

En effet, seul le spermatozoïde capacité peut se lier à la zone pellucide qui entoure l'oeuf et seul le spermatozoïde ayant subi la réaction acrosomale peut pénétrer cette zone pellucide et fusionner avec la membrane de l'oeuf.

La capacitation a lieu in vivo dans le tractus génital de la femme. Ce processus, assez mal connu, semble impliquer une modification des composants de la surface de la tête du spermatozoïde. La réaction acrosomale a lieu in vivo près de l'ovocyte. Elle correspond à une extériorisation de la membrane interne de l'acrosome qui remplace la membrane plasmique de la tête du spermatozoïde et s'accompagne de la perte de la membrane externe et du contenu de l'acrosome.

Le phénomène de capacitation peut être suivi de façon indirecte par le test dit de pénétration des ovocytes dépellucidés de Hamster décrit par Yanagimachi et al (1). Ces bio-essais sont toutefois délicats à mettre en oeuvre. Un inconvénient majeur réside en outre dans le fait qu'une population entière de spermatozoïdes est évaluée sur la performance de quelques spermatozoïdes qui ont pu pénétrer ou fusionner.

Grâce à la microscopie électronique, il a déjà été possible de bien étudier les événements morphologiques exocytotiques correspondant à la réaction acrosomale (2). Cependant, cette technique ne peut être mise en oeuvre pour des observations de routine. En outre, il est difficile d'observer la réaction acrosomale humaine au microscope optique. Jusqu'à présent, seule une technique de triple coloration sur des spermatozoïdes fixés a été proposée à cette fin (3). Une fois encore, ces méthodes s'appliquent très mal à la mise en évidence en routine de la réaction acrosomale chez l'homme.

Etant donné le développement actuel des techniques de fécondation in vitro, il est souhaitable de disposer d'un outil efficace pour suivre la réaction acrosomale et autoriser la mise au point de milieux biologiques qui seraient parfaitement adaptés à la mise en oeuvre de ces techniques.

On a déjà proposé [(4) et (5)] des moyens pour suivre la réaction acrosomale sur des spermatozoïdes viables. Les techniques proposées dans ces références sont des immuno essais qui mettent en oeuvre, comme immunomarqueur, un anticorps monoclonal dressé contre un antigène de la matrice de la membrane acrosomale externe, qui disparaît donc dès que la réaction acrosomale a eu lieu. Ces réactifs sont donc difficilement utilisables en dosages de routine car, expérimentalement, il est beaucoup plus difficile de mettre en évidence un signal qui disparaît qu'un signal qui apparaît.

Il y a donc un besoin réel de disposer d'essais permettant de suivre de façon quantitative et/ou qualitative la réalisation de la réaction acrosomale sur des spermatozoïdes contenus dans un milieu donné.

Dans une publication récente, Fenichel et al (6) ont mis en évidence la capacité d'un anticorps monoclonal de souris dressé contre le trophoblaste humain, appelé GB24, à reconnaître un déterminant antigénique de la région acrosomale de la tête d'un spermatozoïde humain. Ils ont également suggéré que l'antigène reconnu par GB24 était présent sur la membrane acrosomale interne, ce qui laisse entrevoir la possibilité d'utiliser cet anticorps comme marqueur clinique simple et direct de la réaction acrosomale et plus généralement de l'évolution de l'état de maturation d'un spermatozoïde.

La présente invention se propose de fournir d'autres moyens pour suivre la réaction acrosomale sur un spermatozoïde.

En effet, la Demanderesse a maintenant établi que de façon surprenante, le composant C3b du complément ou la molécule inactivée du C3, iC3, ou un fragment de ces protéines ou le composant C3 lui même, est susceptible de se lier au spermatozoïde lorsque celui-ci a subi l'a réaction acrosomale.

C'est pourquoi, l'invention a pour objet un procédé de mise en évidence de la capacité d'un spermatozoïde contenu dans un milieu donné à subir la réaction acrosomale cararactérisé en ce que, sur ledit spermatozoïde sur lequel on induit la réaction acrosomale, on met en évidence qualitativement et/ou quantitativement la fixation de la protéine C3, C3b ou iC3, ou d'un fragment de ces protéines, ajouté(e) au milieu contenant ledit spermatozoïde.

Le complément est un système enzymatique complexe jouant un rôle important sur le mécanisme effecteur de la réponse immunitaire humorale. La protéine C3b est un produit de clivage du complément tandis que la protéine iC3 correspond à une forme du complément ayant perdu ses propriétés fonctionnelles et obtenue, à partir du complément C3, par une suite de congélations et décongélations.

Récemment, de nombreux récepteurs cellulaires spécifiques, soit des formes natives, soit des produits de clivage du complément ont été découverts. Le principe à la base de l'invention est donc la découverte d'un récepteur du composant C3b ou de la protéine iC3 sur les spermatozoïdes.

Bien entendu, tout fragment desdites protéines convient également pour la mise en oeuvre de l'invention, dès lors qu'il se fixe au spermatozoïde.

La mise en évidence de la fixation du C3b ou d'iC3 sur le spermatozoïde peut se faire de différentes façons. Parmi les techniques usuelles, on peut citer en particulier le marquage soit du C3b ou de l'iC3 eux-mêmes, permettant la détection directe de la liaison, soit de préférence d'un anticorps anti-C3, permettant la détection indirecte.

De nombreux anticorps anti-C3, c'est-à-dire dirigés contre le complément sont disponibles dans le commerce sous forme marquée ou non. Il est donc particulièrement intéressant de les mettre en oeuvre, puisqu'ils peuvent reconnaître à la fois la protéine C3b et la protéine iC3. On peut également envisager la préparation d'anticorps monoclonaux dirigés contre les protéines C3b et iC3 elles-mêmes ou leurs fragments qui se fixent au spermatozoïde.

Toutes les techniques de marquage couramment utilisables dans les méthodes de dosage immunologiques peuvent être employées. On peut citer par exemple le marquage par un enzyme, un fluorochrome, un isotope radioactif ou encore une particule. On citera de préférence le marquage par un conjugué fluorescent. Pour procéder à un dosage rapide et peu coûteux de l'activation, on pourra utiliser avec avantage un marquage fluorescent couplé soit à la microscopie à fluorescence, soit à la cytométrie de flux. Cette technique sera décrit plus en détails ci-après.

Parmi les événements précoces de l'activation du spermatozoïde, on constate en particulier l'augmentation du calcium intracellulaire et il est possible d'induire rapidement in vitro la réaction acrosomale par ajout au milieu contenant le spermatozoïde de drogues permettant une entrée massive du calcium à l'intérieur du spermatozoïde. On peut citer à cet égard le produit commercialisé par la société Sigma sous l'appellation ionophore calcique A23187, comme cela a été décrit par Jamil et al (7).

C'est pourquoi, suivant un mode de réalisation avantageux de l'invention, on induit la réaction acrosomale en ajoutant un agent d'induction approprié au milieu contenant je spermatozoïde. De préférence, cet agent est l'ionophore calcique A23187.

Le procédé selon l'invention est particulièrement utile pour suivre la maturation des spermatozoïdes dans un échantillon de sperme donné. Un mode de réalisation avantageux de l'invention consiste à mettre en évidence quantitativement la fixation de la protéine C3, C3b ou iC3 sur des spermatozoïdes contenus dans un échantillon de sperme.

De préférence, on prépare ledit échantillon de sperme à partir d'un sperme humain en séparant les spermatozoïdes du plasma séminal et en sélectionnant les spermatozoïdes viables et mobiles.

Comme indiqué ci-dessus, une technique préférée pour la détermination quantitative de la fixation de la protéine C3, C3b ou iC3 sur un spermatozoïde est la cytométrie de flux.

La cytofluorométrie de flux, du fait qu'elle permet une étude précise des cellules en suspension préalablement marquées par un fluorochrome, permet, outre la détermination quantitative des spermatozoïdes ayant subi la réaction acrosomale, une mesure simultanée du nombre et de la viabilité des spermatozoïdes contenus dans l'échantillon de sperme analysé.

On a constaté que la fixation de la protéine C3b au spermatozoïde est significativement moins bonne lorsque ledit spermatozoïde n'a pas été capacité préalablement à l'induction de la réaction acrosomale. C'est pourquoi, le procédé selon l'invention peut être utilisé pour mettre en évidence l'aptitude d'un spermatozoïde contenu dans un milieu donné à subir l'étape de capacitation. Suivant un mode de réalisation avantageux de l'invention, on induit la capacitation préalablement à l'induction de la réaction acrosomale. A cette fin, on peut utiliser par exemple le milieu B2 de Ménézo, qui permet la survie et la maturation de spermatozoïdes (R. Acad. Spi. Paris ( 1976) 282, ( 1967).

Finalement, il est clair que le procédé conforme à l'invention permet de suivre l'évolution de la maturation d'un spermatozoïde, sous l'effet de différents agents et/ou avec des concentrations variables desdits agents. Il est donc possible de réaliser des spermogrammes permettant d'obtenir des informations précises sur la numération, la viabilité et la capacité à subir la réaction acrosomale de spermatozoïdes.

L'invention a donc également pour objet une trousse pour la réalisation d'un spermogramme caractérisée en ce qu'elle contient la protéine C3, C3b ou iC3 ou un fragment de celles-ci. Bien évidemment, lorsqu'on utilise la cytofluorométrie de flux on visualise et on quantifie la fixation de C3, C3b ou iC3 au spermatozoïde par un anticorps anti-C3 conjugué à un marqueur fluorescent.

Lors de la mise en oeuvre du procédé selon l'invention, les protéines C3, C3b ou iC3 reconnaissent un récepteur sur le spermatozoïde. Ces récepteurs peuvent être obtenus par purification à partir du complexe récepteur-ligand formé avec les protéines C3, C3b ou iC3 ou encore préparés par génie génétique.

3

On peut également préparer des anticorps, monoclonaux ou polyclonaux, dirigés contre ces récepteurs. L'invention a également pour objet ces produits nouveaux.

L'induction de la réaction acrosomale par des produits comme A23187 est efficace. Cependant, ces produits peuvent être toxiques et ne sont donc pas utilisables lorsque les spermatozoïdes activés sont destinés à une fécondation in vitro. Actuellement, le milieu synthétique B2 déjà cité, qui permet la survie et la maturation des spermatozoïdes en quelques heures, est systématiquement utilisé pour les fécondations in vitro. Or, le milieu B2 ne permet l'activation que d'une faible proportion de spermatozoïdes et ne permet pas de déterminer la période optimale d'incubation in vitro des spermatozoïdes avec l'ovule. Il existe donc ici encore, un besoin de disposer d'un milieu de fécondation in vitro permettant une utilisation optimale des spermatozoïdes.

L'invention a donc également pour objet un milieu de fécondation in vitro, caractérisé en ce qu'il contient les protéines C3, C3b ou iC3 ou un fragment de celles-ci ou un anticorps anti-C3, ou un anticorps anti-C3b ou un anticorps anti-iC3.

Plus généralement, le fait que les protéines C3, C3b ou iC3 se lient au spermatozoïde est une indication que ces protéines jouent un rôle dans la régulation de la fécondation du spermatozoïde. On sait par ailleurs, d'après une publication de Fahmi et Hunter, 1985 (8), que la concentration en complément dans le liquide folliculaire est 5 à 20 fois plus élevée que dans le sérum. Dans ce contexte, la découverte à la base de l'invention pourrait permettre de mieux comprendre les interactions oeuf-sperme, et d'agir sur ces interactions, soit in vitro, soit in vivo.

Le composant C3b pourrait se lier de façon covalente à l'oeuf, et la rencontre avec un spermatozoïde activé donnerait lieu à la fécondation à partir de la liaison récepteur-ligand entre le C3b lié à l'oeuf et le récepteur C3b du spermatozoïde. Dans une autre hypothèse, l'oeuf exprime un antigène analogue au composant C3b, et c'est cet antigène qui va se lier au récepteur C3b du spermatozoïde. Par le jeu des liaisons récepteur-ligand, on peut donc prévoir de modifier les interactions oeuf-sperme en les favorisant ou en les inhibant soit dans l'organisme humain, soit in vitro lors de la mise en oeuvre de programmes de fécondation in vitro.

C'est pourquoi, l'invention a également pour objet l'application du récepteur C3, C3b du spermatozoïde, du récepteur iC3 du spermatozoïde, d'anticorps dirigés contre lesdits récepteurs, des protéines C3, C3b ou iC3, ou de fragments de celles-ci, d'anticorps anti-C3 ou plus spécifiquement d'anticorps anti-C3b ou anti-iC3 pour modifier les interactions oeuf/sperme, qu'elles soient in vivo ou in vitro.

Il s'agit tout d'abord de l'utilisation des protéines C3, C3b ou iC3 ou d'anticorps anti-C3 ou du récepteur C3, C3b ou iC3 du spermatozoïde ou d'anticorps dirigés contre le récepteur desdites protéines ou de fragments de celles-ci sur le spermatozoïde pour contrôler l'efficacité ou la rapidité de la fécondation dans les milieux de culture pour fécondation in vitro.

Il s'agit également de l'utilisation du récepteur C3, C3b ou iC3 du spermatozoïde ou d'anticorps anti- C3 comme médicaments ou vaccins contraceptifs à l'usage des hommes ou des femmes.

Il est bien sûr possible d'utiliser à chaque fois des fragments des protéines indiquées, dès lors qu'ils comportent les séquences convenant pour le but indiqué.

L'invention sera mieux comprise à l'aide de la description détaillée ci-après qui comprend un exemple de détermination quantitative de la proportion de spermatozoïdes contenus dans un échantillon de sperme à subir la réaction acrosomale.

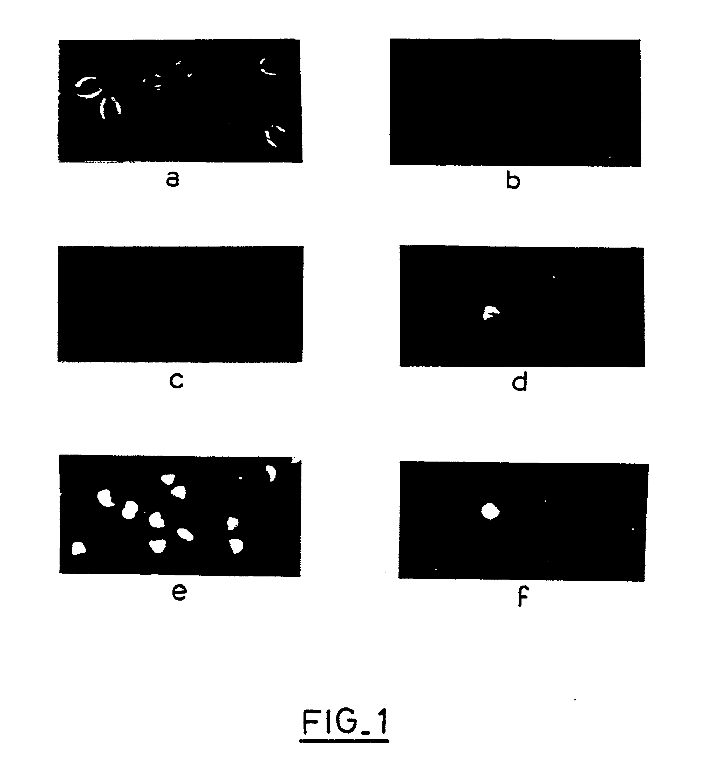

Les figures 1 à 3 suivantes illustrent l'invention :

Figure 1 : elle représente l'analyse de différents échantillons de sperme par immunofluorescence indirecte au moyen du microscope à fluorescence commercialisé par la société Zeiss sous l'appellation Axiophot:

    a) : spermatozoïdes mobiles récemment éjaculés visualisés en contraste de phase ;

    b) : cette photo illustre l'absence de révélation des spermatozoïdes visualisés en a) par le C3b ;

    c) : spermatozoïdes visualisés en contraste de phase pour lesquels la capacitation a été induite pendant 6 heures dans un milieu B2 de Menezo puis la réaction acrosomale induite pendant 1 heure avec 10 μM de A23187 ;

    d) : mêmes spermatozoïdes qu'en c) visualisés par le marquage au C3b ;

    e) spermatozoïdes marqués au C3b après perméabilisation à l'acétone ;

    f) spermatozoïde marqué avec l'anticorps GB24 dans les mêmes conditions qu'en d).

Figure 2 : elle représente sous forme d'un diagramme la relation entre la concentration en A23187 exprimée en μM et la proportion de spermatozoïdes capacités ayant lié soit la protéine C3b, soit GB24.

Figure 3 : elle représente sous forme d'un diagramme la relation entre l'évolution du pourcentage de spermatozoïdes ayant lié GB24 (en abscisse) et l'évolution du pourcentage de spermatozoïdes ayant lié C3b (en ordonnée) pour des concentration variables de A23187.

L'exemple suivant décrit une méthode quantitative de détermination de la proportion de spermatozoïdes contenus dans un échantillon de sperme et ayant subi la réaction acrosomale.

## 1 ° - préparation du sperme

On prélève des échantillons de sperme de donneurs dont la fertilité est établie ou d'hommes participants à un programme de fécondation in vitro, le sperme prélevé répondant aux conditions standards suivantes : plus de $50 \times 10^6$ spermatozoïdes/ml ; plus de 70 % de spermatozoïdes mobiles ; plus de 70 % de forme normale. On récupère les spermatozoïdes viables et mobiles en utilisant la technique dite de "swim-up". Selon cette technique, on liquéfie le sperme pendant 30 mn à 37°C, puis on le lave deux fois dans un milieu Tyrode (commercialisé par la société Eurobio, Paris, France) puis on centrifuge à 500 g pendant 10 mn, puis on procède au "swim-up", c'est-à-dire qu'on dépose sur le culot de spermatozoïdes du milieu B2 de Ménézo qui contient 1 % de sérum albumine bovine et 1.75 mM de $CaCl_2$ puis on récupère à partir du surnageant une fraction enrichie de spermatozoïdes viables et mobiles dont on ajuste la concentration finale des spermatozoïdes dans le milieu B2 aux environs de 1 à $10 \times 10^6$/ml. On détermine la mobilité et la viabilité par microscopie en contraste de phase et par exclusion au bleu de Trypan. On place une partie des échantillons sur des plaques, on les sèche à l'air et on les fixe ensuite à l'acétone pendant 10 mn.

## 2° - Induction de la capacitation et de la réaction acrosomale

on met à incuber les spermatozïdes mobiles et viables dans un milieu B2 à 37°C sous 5 % de $CO_2$ et 95 % d'air pendant 6 heures pour induire la capacitation. Ensuite, on induit la réaction acrosomale en mettant à incuber les spermatozoïdes avec l'ionophore calcique A23187, de façon à atteindre des concentrations finales dans le milieu B2 de 1 à 20 micromoles, pendant des périodes de 30, 60 et 120 mn.

## 3° : Préparation du complément

On obtient le composant C3b du complément en traitant le complément C3 commercialisé par la Société SIGMA avec de la trypsine dans les conditions suivantes : 1 mg de trypsine pour 1 g de C3, pendant 1 heure à température ambiante. Ensuite, la réaction est arrêtée par l'addition de 2 mg d'inhibiteur de trypsine de soja commercialisé par la Société SIGMA. La protéine iC3 est obtenue après une série de 5 congélations dans l'azote liquide et de décongélations du complément C3.

## 4° - Méthode de détection par immunofluorescence

a) immunofluorescence indirecte :
On met à incuber les spermatozoïdes avec le composant C3b ou iC3 dans un milieu B2 pendant 30 mn à raison de 5 µg de C3b ou d'iC3 pour 100 µl d'une suspension contenant $10^6$ spermatozoïdes, puis on lave le milieu avec un tampon PBS, 0,15 M, pH 7, puis le milieu est mis à incuber avec des anticorps anti-C3 obtenus chez la chèvre. Ces anticorps sont ensuite révélés par un anticorps de lapin anti-immunoglobulines de chèvre conjugué au FITC en utilisant la méthode de Johnson & Holborow (9), de façon à obtenir un rapport final FITC/anticorps de 2:1. Après lavage final avec un tampon PBS, on examine les échantillons avec un microscope à fluorescence commercialisé par la Société Zeiss sous l'appellation Axiophot. On fixe certains échantillons des suspensions de sperme avec du formaldéhyde (1 % dans PBS) et on les stocke à 4°C.
b) cytométrie de flux :
On utilise l'appareil commercialisé sous la désignation ATC3000 par la société ODAM, Wissembourg, France, pour analyser les suspensions de sperme obtenues après incubation dans le milieu B2 et différencier les cellules devenues fluorescentes de l'ensemble de la population cellulaire.

On part d'échantillons contenant $5 \times 10^6$ spermatozoïdes/ml de PBS. On établit une "fenêtre de sperme", conformément à la technique décrite par Haas et Cunningham (10), en utilisant une distribution des spermatozoïdes selon la dispersion de la lumière, soit dans le prolongement du faisceau (correspondant à la taille des cellules), soit suivant un axe perpendiculaire au faisceau (correspondant à la densité des cellules).

Pour chaque échantillon, on compte à l'intérieur de la "fenêtre de sperme", par cytométrie, l'intensité de fluorescence de 10 000 cellules. On se fixe un seuil de façon à ce que les spermatozoïdes dont l'intensité de fluorescence est en deçà de ce seuil soient considérés comme négatifs et non représentés.

Les résultats de l'analyse par cytométrie de flux, illustrés par la figure 1, permettent de conclure que la protéine C3b ne réagit pas avec des spermatozoïdes récemment éjaculés et séparés du liquide séminal (Fig. 1b). Par contre, lorsque les spermatozoïdes sont d'abord fixés puis perméabilisés à l'acétone, on observe une réaction sur la région acrosomale de la tête du spermatozoïde (Fig. 1e) ce qui prouve que le récepteur C3b du spermatozoïde se trouve effectivement dans la région acrosomale. Environ 40 % des spermatozoïdes mobiles et viables préparés comme indiqué précédemment réagissent positivement après capacitation et induction de la réaction acrosomale par incubation avec A23187 (10 µM dans le milieu B2, 1/200 ethanol, 1 heure). Par comparaison, on a représenté sur la figure 1f les résultats obtenus avec GB24 qui reconnaît une région identique de la tête du spermatozoïde.

Par ailleurs, une analyse quantitative par cytométrie de flux de la réactivité de la protéine C3b et de la protéine iC3 avec le spermatozoïde à différents stades de maturation a donné les résultats indiqués dans le tableau I ci-après, qui compare les résultats obtenus avec C3b à ceux obtenus avec GB24.

## TABLEAU I

| Etat de maturation des spermatozoïdes | % de spermatozoïdes ayant réagi avec | C3b | iC3 | GB24 |
|---|---|---|---|---|
| avec capacitation<br>- sans A23187<br>- avec A23187 | | 10<br>41 | 6<br>32 | 5<br>43 |

Il résulte clairement de ce tableau que des réactions analogues sont obtenues avec C3b, iC3 et GB24. La plus grande quantité de spermatozoïdes ayant réagi est obtenue lorsque les spermatozoïdes se trouvent dans leur état de maturation le plus avancé, c'est-à-dire après capacitation et induction de la réaction acrosomale avec A23187.

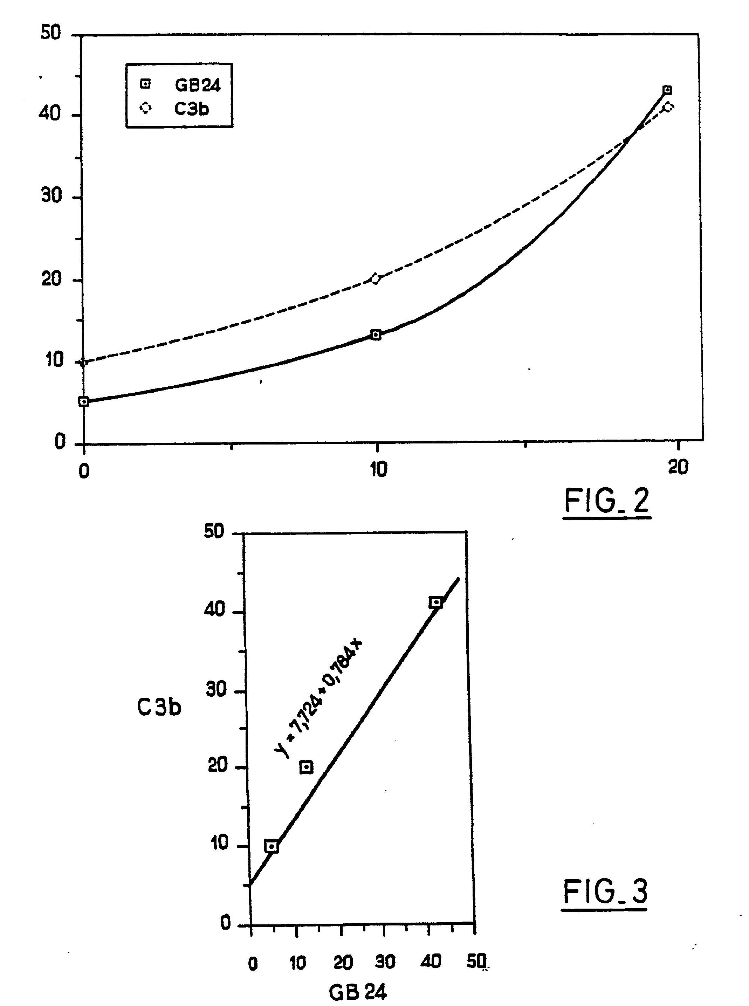

On constate en outre que la réactivité du composant C3b est dépendante de la concentration en A23187. La figure 2 montre qu'une réponse optimale est obtenue avec une concentration de 20 µM. Les résultats sont analogues à ceux obtenus avec GB24. En outre, pour chaque concentration de A23187, on a mesuré le pourcentage de spermatozoïdes ayant lié C3b ou GB24 (Fig. 3) et on a établi que si on cherche à représenter l'évolution du pourcentage de spermatozoïdes ayant lié le C3b avec l'évolution du pourcentage de spermatozoïdes ayant lié GB24, on obtient une corrélation linéaire, ce qui signifie que, tout comme GB24, C3b est un marqueur de la réaction acrosomale sur les spermatozoïdes humains.

## REFERENCES

(1) YANAGIMACHI R., YANAGIMACHI H. & ROGERS B.J. (1976) Biol. Reprod. 15 : 471-476

(2) BARROS C., BEDFORD, J.M., FRANKLIN M.C. & AUSTIN, C.R. (1967) J. Cell Biol. 34, 1-5

(3) TALBOT, P. & CHACON, R.S. (1981) J. Exp. Zool. 215, 201-208

(4) BYRD, W. & WOLF, D.P. (1986) - Biol. Reprod. 34, 859-869

(5) KALLAJOKI, M., VIRTANEN, I. & SUOMINEN, J. (1986) - Int. J. Androl. 9, 181-194

(6) FENICHEL, P., HSI, B.L., FARAHIFAR, D., DONZEAU M., BARRIER-DELPECH D., et YEH C.J.G. (1989) - J. Reprod. Fert. 87, 699-706.

(7) JAMIL, K. et WHITE, I.G. (1981) Archs. Androl. 7, 283-292

(8) FAHMI et HUNTER (1985)

(9) JOHNSON, G.D. & HOLBOROW, E.J., (1986) - Handbook of Experimental 4e édition, p. 28.4-28.5

(10) HAAS, G.G. & CUNNINGHAM, M.S. (1984) - Fert. Steril. 42, 606-613.

## Revendications

1. Procédé de mise en évidence de la capacité d'un spermatozoïde contenu dans un milieu donné à subir la réaction acrosomale, caractérisé en ce que, sur ledit spermatozoïde sur lequel on induit la réaction acrosomale, on met en évidence qualitativement et/ou quantitativement la fixation de la protéine C3, C3b ou iC3 ou d'un fragment de ces protéines, ajouté(e) au milieu contenant ledit spermatozoïde.

2. Procédé selon la revendication 1, caractérisé en ce que la protéine ajoutée est marquée.

3. Procédé selon la revendication 1, caractérisé en ce qu'on ajoute au milieu un anticorps anti-C3 marqué.

4. Procédé selon l'une des revendications 2 ou 3, caractérisé en ce que le marquage est effectué à l'aide d'un conjugué fluorescent.

5. Procédé selon l'une des revendications 1 à 4, caractérisé en ce que l'induction de la réaction acrosomale est effectuée par ajout au milieu contenant le spermatozoïde d'un agent d'induction approprié.

6. Procédé selon l'une des revendications 1 à 5, caractérisé en ce que ledit agent est l'ionophore calcique A23187.

7. Procédé selon l'une des revendications 1 à 6, caractérisé en ce que avant induction de la réaction acrosomale, ledit spermatozoïde est capacité.

8. Procédé selon l'une des revendications 1 à 7, caractérisé en ce qu'on met en évidence quantitativement la fixation de la protéine ajoutée sur des spermatozoïdes contenus dans un échantillon de sperme.

9. Procédé selon la revendication 8, caractérisé en ce que ledit échantillon de sperme est obtenu à partir d'un sperme humain en séparant les spermatozoïdes du plasma séminal et en sélectionnant les spermatozoïdes viables et mobiles.

10. Procédé selon la revendication 9, caractérisé en ce que la quantification de la fixation se fait par cytométrie de flux.

11. Procédé selon l'une des revendications 1 à 10, caractérisé en ce qu'on induit la capacitation préalablement à l'induction de la réaction acrosomale.

12. Application du procédé selon l'une des revendications 1 à 11, à la mise en évidence de l'aptitude d'un spermatozoïde contenu dans un milieu donné à subir l'étape de capacitation.

13. Trousse pour la mise en oeuvre du procédé selon l'une des revendications 1 à 11, notamment pour la réalisation d'un spermogramme, caractérisée en ce qu'elle contient les protéines C3, C3b ou iC3 ou un fragment de celles-ci.

14. Milieu de culture convenant pour la fécondation in vitro, caractérisé en ce qu'il contient les protéines C3, C3b ou iC3 du complément ou un fragment de ces protéines, ou portant ces protéines, ou un anticorps dirigé contre lesdites protéines ou un fragment de celles-ci.

15. Le récepteur C3b du spermatozoïde.

16. Un anticorps dirigé contre le récepteur décrit dans la revendication 15.

17. Le récepteur iC3 du spermatozoïde.

18. Un anticorps dirigé contre le récepteur décrit dans la la revendication 17.

19. Utilisation des protéines C3, C3b ou iC3 ou d'un fragment desdites protéines ou de protéines portant C3, C3b ou iC3, d'anticorps dirigés contre lesdites protéines ou l'un de leurs fragments, des produits selon l'une quelconque des revendications 15 à 18, pour modifier les interactions oeuf/sperme.

**20.** A titre de médicaments, les produits selon l'une des revendications 15 ou 16, ou, les, anticorps anti-C3.

**21.** A titre de vaccins contraceptifs à l'usage des hommes ou des femmes, les produits selon l'une des revendications 15 ou 16 ou les anticorps anti-C3.

**22.** Milieu de culture convenant pour la fécondation in vitro, caractérisé en ce qu'il contient le récepteur C3 ou le récepteur iC3 ou le récepteur iC3 du spermatozoïde ou un anticorps dirigé contre lesdits récepteurs.

FIG_1

FIG. 2

FIG. 3

Office européen
des brevets

**RAPPORT DE RECHERCHE EUROPEENNE**

Numero de la demande

EP 91 40 0748

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | EP-A-0 241 444 (U.R. NILSSON et al.) * Page 8, ligne 30 - page 9, ligne 24 * --- | 20 | G 01 N 33/68 C 12 N 5/08 C 07 K 15/06 C 07 K 15/28 A 61 K 37/02 A 61 B 17/425 |
| A | J. REPROD. FERT., vol. 87, 1989, pages 699-706; P. FENICHEL et al.: "Evaluation of the human sperm acrosome reaction using a monoclonal antibody, GB24, and fluorescence-activated cell sorter" * Pages 699-700 * --- | 1,4-12 | |
| A | PROC. NATL. ACAD. SCI (USA), vol. 82, février 1985, pages 859-863; J.L. COLE et al.: "Identification of an additional class of C3-binding membrane proteins of human peripheral blood leukocytes and cell lines" * Résumé * --- | 15-17, 19-21 | |
| A | CHEMICAL ABSTRACTS, vol. 104, no. 23, 9 juin 1986, page 556, résumé no. 205057m, Columbus, Ohio, US; T.F. SCHULZ et al.: "Enzyme-linked immunoassay to monitor the purification of, and to screen for monoclonal antibodies against, C3b receptor", & COMPLEMENT 1986, 2(4), 219-229 * Résumé * --- | 15-21 | **DOMAINES TECHNIQUES RECHERCHES (Int. Cl.5)** G 01 N C 07 K C 12 P C 12 N |
| A | C.R. ACAD. SC. PARIS, vol. 282, série D, 14 juin 1976, pages 167-170; M.Y. MENEZO: "Milieu synthétique pour la survie et la maturation des gamètes et pour la culture de l'oeuf écondé" * En entier * ---                      -/- | 22 | |

Le présent rapport a été établi pour toutes les revendications

| Lieu de la recherche | Date d'achèvement de la recherche | Examinateur |
|---|---|---|
| LA HAYE | 18-06-1991 | HITCHEN C.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

....................................................................
& : membre de la même famille, document correspondant

EPO FORM 1503 03.82 (P0402)

Page 2

**Office européen des brevets**

# RAPPORT DE RECHERCHE EUROPEENNE

Numero de la demande

EP 91 40 0748

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A,P | WO-A-9 009 802 (CENTER FOR INNOVATIVE TECHNOLOGY)<br>* Abrégé; pages 7-10 * | 21 | |
| | | | **DOMAINES TECHNIQUES RECHERCHES (Int. Cl.5)** |

Le présent rapport a été établi pour toutes les revendications

| Lieu de la recherche | Date d'achèvement de la recherche | Examinateur |
|---|---|---|
| LA HAYE | 18-06-1991 | HITCHEN C.E. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P0402)